# EUROPEAN PATENT SPECIFICATION

(11) **EP 2 543 345 B1**
(45) Date of publication and mention of the grant of the patent: **22.08.2018**
(21) Application number: 11750177.5
(22) Date of filing: 02.03.2011
(51) Int. Cl.: A61F 2/86, A61F 2/06, A61B 17/12, A61F 2/90

(54) **SURGICAL APPARATUS FOR ANEURYSMS**
CHIRURGISCHE VORRICHTUNG FÜR ANEURYSMEN
INSTRUMENT CHIRURGICAL POUR LES ANÉVRISMES

(30) Priority: 02.03.2010 CN 201010116448
(43) Date of publication of application: 09.01.2013
(73) Proprietor: MicroPort NeuroTech (Shanghai) Co., Ltd., Shanghai 201318 (CN)
(72) Inventor: JIN, Qiaorong, Shanghai 201203 (CN); LI, Yu, Shanghai 201203 (CN); WANG, Sen, Shanghai 201203 (CN); XIE, Zhiyong, Shanghai 201203 (CN); LUO, Qiyi, Shanghai 201203 (CN)
(74) Representative: Dennemeyer & Associates S.A.
(86) International application number: PCT/CN2011/071447
(87) International publication number: WO 2011/107024

(56) References cited:
- EP-A1- 2 208 483
- EP-A1- 2 532 381
- EP-A2- 1 095 634
- WO-A2-2007/095031
- WO-A2-2007/139698
- WO-A2-2009/042789
- CN-A- 101 472 536
- CN-U- 201 625 318
- CN-Y- 2 889 337
- CN-Y- 201 379 671
- US-A1- 2002 177 870
- US-A1- 2009 270 974
- US-A1- 2009 270 974
- US-A1- 2010 004 730
- US-B2- 7 655 031

## Description

### TECHNICAL FIELD

The present application relates to medical instrument, in particular to a surgical apparatus for aneurysms.

### BACKGROUND ART

The wall of an arterial vessel becomes weak locally due to diseases, injuries or congenital factors of it. Struck by blood flow, a weak point of the arterial vascular wall protrudes outward and dilates gradually, and thus forms an aneurysm. Aneurysms occur in different parts of the body. Abdominal aortic aneurysm and intracranial aneurysm are most common. What's fundamental in aneurysm treatments which aim at reducing the risk of aneurysmal rupture is to achieve healing of the parent artery and reconstruction of an anatomical structure of the arterial wall.

Current endovascular intervention for aneurysms mainly uses the method of stent-assisted coiling, i.e., delivering a stent of appropriate density to the pathologically changed blood vessel, and then delivering the coil through a pore of the stent to the aneurysm, to achieve the goal of treatment by filling the aneurysm.

As the terminal action of an aneurysm embolization occurs in the aneurysm cavity, by studying the prior art, the applicants have found: during the process of treating aneurysm with a stent-assisted coil currently available, the coil shows a mass effect as evidenced by symptoms of compression of the peritumoral brain tissue, vital blood vessels and nerves; meanwhile, the fully dense occlusion rate of coil filling is low, and the postoperative recurrence is high. In addition, the head end of the coil can pierce a thin aneurysmal wall easily, which will induce aneurysm rupture and lead to intraoperative or postoperative death of a patient directly.

Document US 2010/0004730 A1 describes a surgical apparatus suitable for delivering a stent to a human body. The apparatus comprises a delivery guide wire, an introducer sheath and a microcatheter. The delivery guide wire is provided with markings fixed on the distal end of an inner core as visual indications for the delivery process.

Document WO 2007/139698 A2 also relates to a surgical apparatus suitable for delivering a stent to a human body with the aid of a delivery guide wire. Delivery positioning elements are fixed on a metal core of the delivery guide wire. EP 2 532 381 is prior art under Article 54(3) EPC and discloses a medical guide wire for delivering an implant instrument, comprising: a metal core, a coil, a delivery element and a boss.

### SUMMARY OF THE INVENTION

In view of the above technical problems, examples of the present application provide a surgical apparatus for aneurysms with the following technical solutions:
a surgical apparatus for aneurysms, comprising: a stent, a delivery guide wire, an introducer sheath and a microcatheter, wherein
the said stent is self-expanding;
the delivery guide wire is placed in an inner cavity of the said introducer sheath with the stent restrained on the outside of it; and
the introducer sheath is connected with the microcatheter with lumens communicating to form a passageway through which the delivery guide wire and the stent are deliverable into a human body.

The delivery guide wire comprises:
a metal core for delivering and supporting the stent;
a spring element covering the metal core;
a boss fixed on the metal core, for providing a pushing force for the stent during delivery; and
a plurality of delivery positioning elements fixed on the external surface of the spring element, for providing pushing or withdrawing forces for the stent during delivery.

Preferably, the self-expanding stent is woven with biocompatible metal filaments and/or polymer filaments.

Preferably, the self-expanding stent is in a mesh tube structure.

Preferably, the mesh tube structure has a compression ratio in the range of 1:2 to 1:10 in the radial direction.

Preferably, the mesh structure is a uniform lattice structure.

Preferably, the uniform lattice structure has a coverage rate in a range of 20% to 60%.

Preferably, the uniform lattice structure has a coverage rate in a range of 30% to 50%.

Preferably, the mesh tube structure as a lattice structure is non-uniform in the axial and/or the radial direction at the site of an aneurysm, but is uniform in the rest parts.

Preferably, the non-uniform lattice structure has a coverage rate in a range of 40% to 60%.

Preferably, the uniform lattice structure has a coverage rate in a range of 20% to 40%.

Preferably, materials of the spring element, the boss and the delivery element are visualizable materials.

Preferably, the introducer sheath is in a hollow structure.

Preferably, the material of the introducer sheath is a polymeric material.

Preferably, the polymeric material is PTFE material, HDFE material or FEP material.

Preferably, the microcatheter comprises:
a tube body in a step-like hollow structure with its diameter and hardness decreasing gradually from the proximal end to the distal end;
a stress dispersion tube with one end connected with the tube body to prevent the tube body from zigzagging at its proximal end; and
an adapting piece used to connect the introducer sheath with the tube body, which is connected with the other end of the stress dispersion tube and has the introducer sheath being inserted therein.

Preferably, the tube body is made of the following materials from inside to outside: a polymeric material for a smooth layer, metals and/or polymers for a reinforcement layer and a polymeric material for a jacket layer.

Preferably, the distal end of the tube body is further provided with a visualization element, for indicating the position of the microcatheter in a blood vessel.

As can be seen from the above technical solutions provided in the examples of the present application, the stent of the aneurysm surgical apparatus in the examples of the present application has a high-density lattice structure and thus a high coverage rate. Especially, due to the non-uniform lattice structure on the stent with a high coverage rate adjacent to the aneurysm, it is like that the released stent has reconstructed the arterial wall at the site of the vascular lesion so that the direction of the blood flow at the site can be significantly changed. As a result, blood strikes on the inner wall of the aneurysm have been avoided leading to an achievement of the purpose of the vascular aneurysm treatment. Meanwhile, dense mesh filaments of the stent, serving as a support for the growth or migration of the vascular endothelial cells, accelerate the growth of intima adjacent to an orifice of the aneurysm, so that the blood vessel at the lesion site can be re-covered by intima, thereby achieving a real anatomical cure of aneurysm.

Regarding the aneurysm surgical apparatus in the examples of the present application, the stent is restrained on the delivery guide wire, and the stent and the delivery guide wire are pre-mounted into the introducer sheath. During a surgical delivery, first, the microcatheter is inserted into the pathologically changed blood vessel and then, the introducer sheath is connected to the microcatheter. After that, by applying a force to the delivery guide wire in an axial direction, the stent restrained on the delivery guide wire is fed from the introducer sheath into the microcatheter and moved to the vascular lesion. At the end, the stent is positioned and released at the site of the vascular lesion by adjusting relative positions between the delivery guide wire and the microcatheter.

In addition, when the stent of the aneurysm surgical apparatus in the examples of the present application is delivered to and released at the site of the vascular lesion, it can further serve as a support or a shield for the embolization substance (e.g., a detachable coil, embolic liquid, etc.) in an aneurysm. This will ensure that the embolization material is maintained in the aneurysm only, to keep the parent artery open and to assist the treatment of vascular aneurysm.

### BRIEF DESCRIPTION OF THE DRAWINGS

Below are provided brief introductions to the figures used to illustrate the technical solutions in the examples of the present application or the prior art. Obviously, figures in the following description are merely examples recorded in the present application.
Fig. 1 is a diagram of the structure of the aneurysm surgical apparatus provided in the examples of the present application;
Fig. 2 is a local section view of the aneurysm surgical apparatus provided in the examples of the present application;
Fig. 3 is a diagram of the structure of the stent in the examples of the present application;
Fig. 4 is a diagram showing the compression of the stent in the examples of the present application;
Fig. 5 is a plane diagram showing the mesh tube structure of the stent in the examples of the present application;
Fig. 6 is a diagram showing the structure of the delivery guide wire in the examples of the present application;
Fig. 7 is a diagram showing the structure of the boss and the delivery elements of the delivery guide wire in the examples of the present application;
Fig. 8 is a diagram of the structure of the microcatheter in the examples of the present application;
Fig. 9 is a diagram showing a stent in the examples of the present application that is delivered to the site of the vascular lesion;
Fig. 10 is a diagram showing how the stent in the examples of the present application is released in a pushing-and-withdrawing way; and
Fig. 11 is a diagram showing how the stent in the examples of the present application is released in a withdrawing-and-pushing way.

### DETAILED DESCRIPTION OF THE INVENTION

The most fundamental method of treating an aneurysm is to achieve a healing of the parent artery and reconstruction of the anatomical structure of the arterial wall. However, current endovascular intervention therapy of surgical stent-assisted coiling presents mass effect, non-dense embolization, and risks of intraoperative or postoperative aneurysm rupture during the treatment of aneurysms.

Examples of the present application provide a surgical apparatus for aneurysms, which can deliver a stent of high density and extreme softness to the site of the vascular lesion and release it. The lattice structure of the stent at the vascular lesion site has a high coverage rate providing to the stent released into the blood vessel an effect as of the parent artery has been healed and thus making a better vascular aneurysm treatment.

Above are core ideas of the present application. To ensure that the skilled in the art understand the technical solutions of the present application better, clear and complete descriptions of the technical solutions in the examples are provided as follows in connection with figures in the examples of the present application. Obviously, the described examples are only part instead of all of the examples of the present application. Based on the examples of the present application, all the other examples obtained by the skilled in the art without inventive efforts should fall within the protection scope of the present application.

The examples of the present application provide a surgical apparatus for aneurysms.

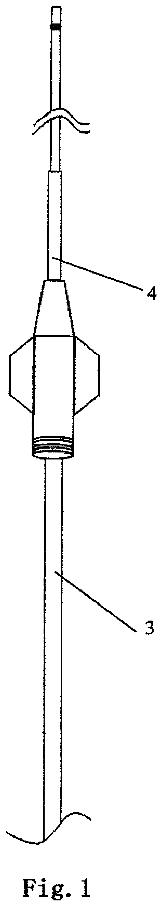

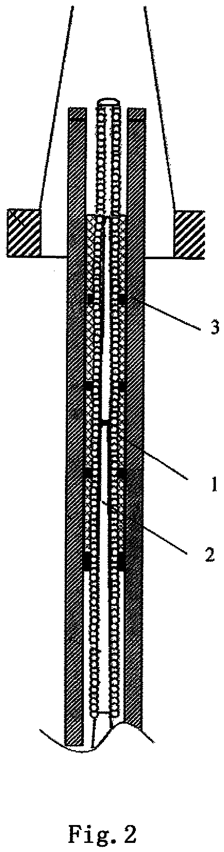

Fig. 1 is the diagram of the outer structure of the aneurysm surgical apparatus; Fig. 2 is the local section view of the surgical vascular apparatus. Combining Figs. 1 and 2, the aneurysm surgical apparatus comprises: a stent (1), a delivery guide wire (2), an introducer sheath (3) and a microcatheter (4).

The stent (1) used to support the pathologically changed blood vessel is restrained on the outside of the distal end of the delivery guide wire (2); the delivery guide wire (2) is provided in the introducer sheath (3) for delivering the stent; the introducer sheath (3) is used for pre-mounting the stent (1) and the delivery guide wire (2). The distal end of the importing sheath (3) is connected with the microcatheter (4) to allow the delivery guide wire (2) and the stent (1) entering into the microcatheter (4); and the microcatheter (4) is used for providing the delivery guide wire (2) and the stent (1) with a passageway into the pathologically changed blood vessel during delivery.

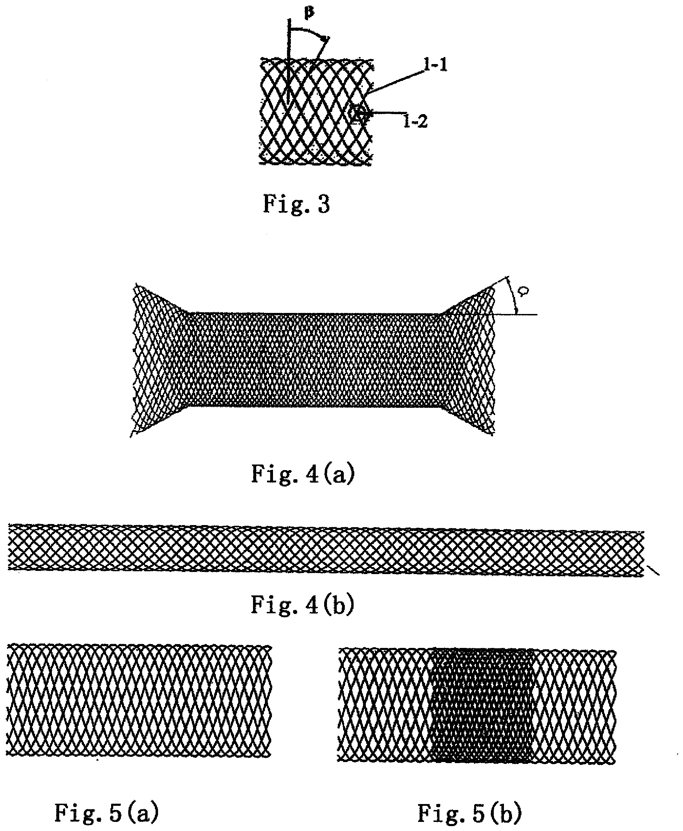

The stent (1) is a highly soft and flexible self-expanding stent having a continuous mesh tube structure with high density. The stent (1) is woven with biocompatible metal filaments and/or polymer filaments. As shown in Fig. 3, each filament of the mesh tube structure is at a braiding angle β relative to the radial direction in a range of 15 degree to 85 degree to ensure that the stent (1) has enough supporting force on radial and circular directions. As shown in Fig. 3, filament (1-1) continuous in the axial direction is rotatable around the filament braiding point (1-2) which serves as a center. This provides sufficient flexibility to the stent (1) and enables its bending or twisting in three dimensions. Therefore, when released into a blood vessel, the stent which will be in a shape more similar to that of the vessel can conform to the tortuous cerebral vessel and prop up the lumen morphology at the same time. As shown in Fig. 4, the variable structure mentioned above further provides the stent (1) with a high compressible property which can be represented by a compression ratio up to from 1:2 to 1:10. The compressed stent (1) can be packed into the introducer sheath or the microcatheter with a diameter of 0.3 mm to 1.5 mm.

The mesh tube structure of the stent (1) can be completely uniform and continuous lattices with a coverage rate in a range from 20% to 60% as shown in Fig. 5(a). In the examples of the present application, a coverage rate of the uniform and continuous lattices from 30% to 50% is chosen. Further, the mesh tube structure of the stent (1) as a lattice structure can be non-uniform in the axial and/or the radial direction at the site of an aneurysm, but be uniform in the rest parts. As shown in Fig. 5(b), after the non-uniform and continuous lattices are delivered into the blood vessel to a region on or near the orifice of the aneurysm, this region would have the highest coverage rate of up to 40% to 60%. Such a high coverage rate can change blood flow in the aneurysm to the greatest extent. The uniform and continuous lattices in the rest parts have a lower coverage rate in a range between 20% to 40%. This can provide sufficient supports to normal vascular walls adjacent to the aneurysm to maintain patency of the parent artery lumen. Meanwhile, this has also reduced the coverage of the lattices to the parent artery branchs to a best extent to minimize their impact on blood flow from the parent artery to the branchs.

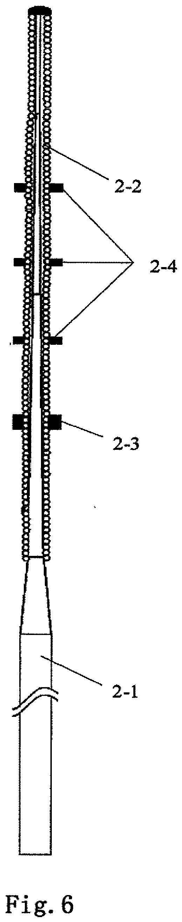

As shown in Fig. 6, the delivery guide wire (2) comprises: a metal core (2-1), a spring element (2-2), a boss (2-3) and a plurality of delivery positioning elements (2-4), wherein the structure of the metal core (2-1) from the proximal end to the distal end is straight-thread-like, step-like with gradually decreasing diameter and then straight-thread-like again. It is used for delivering and supporting the stent (1). The spring element (2-2) covers the straight-thread-like structure at the distal end and the step-like structure in the middle of the metal core (2-1). The boss (2-3) is fixed on the metal core (2-1) for providing the stent (1) with a pushing force during delivery; and the plurality of delivery positioning elements (2-4) are fixed on the external surface of the spring element (2-2) or the metal core (2-1) and positioned in front of the boss (2-3) for providing pushing or withdrawing forces for the stent during delivery.

The material for metal core (2-1) can be selected from stainless steel, nickel-titanium alloy, copper alloy, aluminum alloy, etc. Moreover, the metal core can be made by grinding one material, as well as by bonding or welding two materials. In accordance with vascular tortuosity, the core's diameter usually reduces gradually from a diameter range of 0.025 inches to 0.012 inches (0.635-0,305mm) of the straight-thread-like structure at the proximal end to a range of 0.012 inches to 0.002 inches (0,305-0,051mm) of the straight-thread-like structure at the distal end. The straight-thread-like structure at the proximal end can have a length ranging from 1500 mm to 2000 mm, the step-like structure in the middle can have a length ranging from 300 mm to 500 mm, and the straight-thread-like structure in the distal end can have a length ranging from 10 mm to 30 mm.

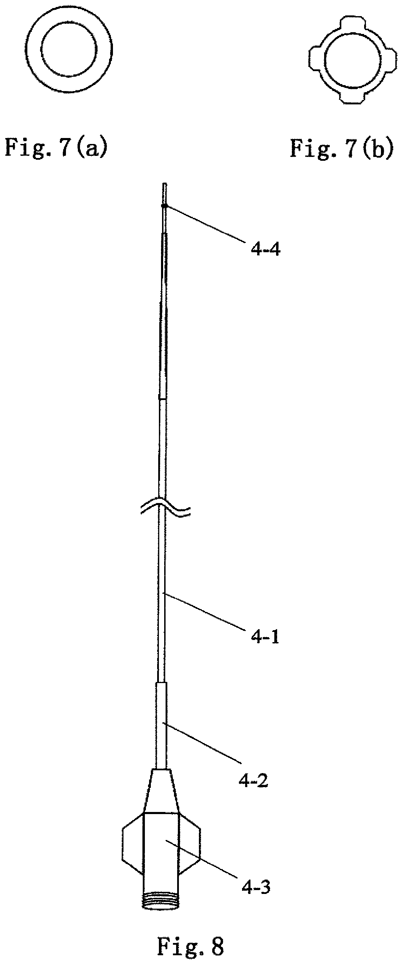

As shown in Fig. 7(a), the boss (2-3) is in a structure of a metal ring sheet. As shown in Fig. 7(b), the shape of delivery elements (2-4) has four peripheral polygons with smooth corners. The number of the delivery element depends on the length of the stent (1). During a delivery, the delivery elements (2-4) drag the stent (1) forward and/or backward via frictions between the corners and the lattices of the stent (1) and/or insertion of the corners into the lattices of the stent (1). In the examples of the present application, the number of the delivery element is four.

The materials of the spring element (2-2), the boss (2-3) and the delivery positioning elements (2-4) can be selected from visualizable materials such as tantalum, platinum, gold, tungsten or polymers.

The introducer sheath (3) is a polymeric tube in a hollow structure with low frictional coefficient. Its material can be PTFE material, HDFE material, FEP material, etc. Stent (1), which is compressed and restrained on the delivery guide wire (2), is usually pre-mounted in the introducer sheath (3). During a delivery, the delivery guide wire (2) is used to help push the stent (1) from the introducer sheath (3) into the microcatheter (4).

As shown in Fig. 8, the microcatheter (4) comprises: a tube body (4-1), a stress dispersion tube (4-2), an adapting piece (4-3) and a visualization element (4-4), wherein the tube body (4-1) is in a step-like hollow structure with its diameter and hardness gradually decreasing from the proximal end to the distal end. The stress dispersion tube (4-2) has one end connected with the tube body (4-1) to prevent the tube body (4-1) from zigzagging or bending at its proximal end. The adapting piece (4-3) used to connect the introducer sheath (3) with the tube body (4-1) is connected with the other end of the stress dispersion tube (4-2) and has the introducer sheath (3) being inserted in it. The visualization element (4-4) is provided at the distal end of the tube body (4-1) for indicating the position of the microcatheter in a blood vessel during the surgery.

The tube body (4-1) has different structures, hardness and diameters along the axial direction in accordance with the vascular tortuosity and size of a vessel, wherein its structure is straight-thread-like, step-like and straight-thread-like sequentially from the proximal end to the distal end, with a length range of 80 cm to 160 cm, 20 cm to 40 cm and 4 cm to 8 cm, respectively. The tube body is of single cavity and comprises multiple layers, namely a smooth layer composed of polymeric materials, a support reinforcement layer made by weaving and/or twisting metals and/or polymers and a jacket layer made by extruding or bonding polymeric materials of different hardness along a hardness gradient from inside to outside.

The aneurysm surgical apparatus in the examples of the present application is used for intracranial aneurysm surgery. The skilled in the art should know that by only changing the size, this aneurysm surgical apparatus can further be applied to abdominal aneurysm surgery or aneurysm surgeries for other parts of the body. These modifications should also be considered as within the protection scope of the present application.

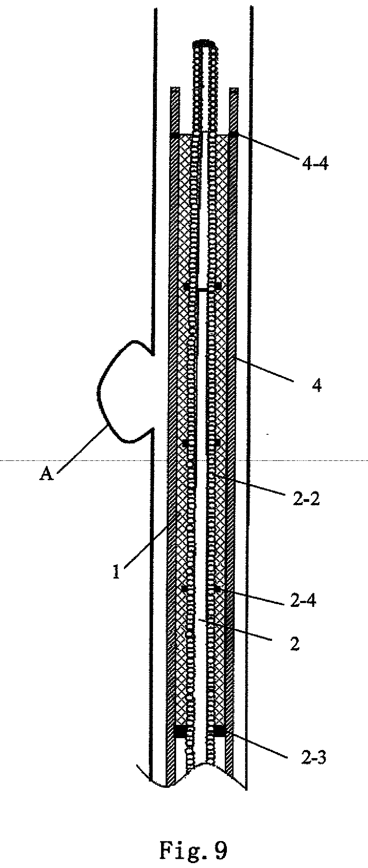

During a delivery in an aneurysm surgery performed with the said aneurysm surgical apparatus, first, the microcatheter (4) is fed from a surgical wound into the blood vessel, then the distal end of the tube body (4-1) of microcatheter (4) is delivered to be close to the vascular lesion site according to the position indicated under X-rays by the visualization element (4-4) on the microcatheter. The stent (1) bound to the delivery guide wire (2) and compressed in the introducer sheath (3) is fed into the microcatheter by the application of an axial force to the delivery guide wire (2). Following positions of the spring element (2-2), the boss (2-3) and the delivery positioning elements (2-4) on the delivery guide wire (2) visualized under X-rays, the stent (1) is navigated to the vascular lesion site as shown in Fig. 9.

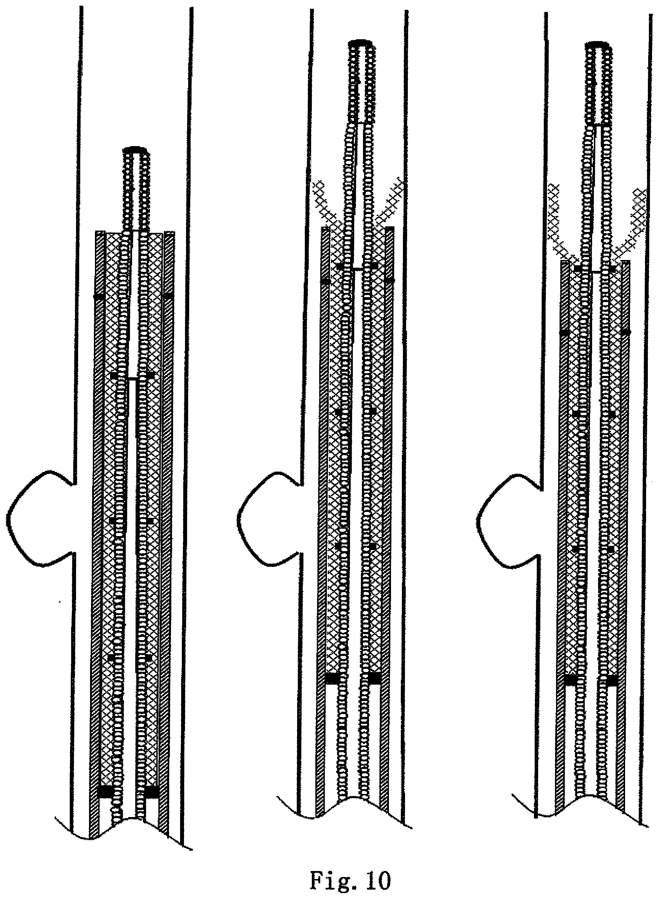

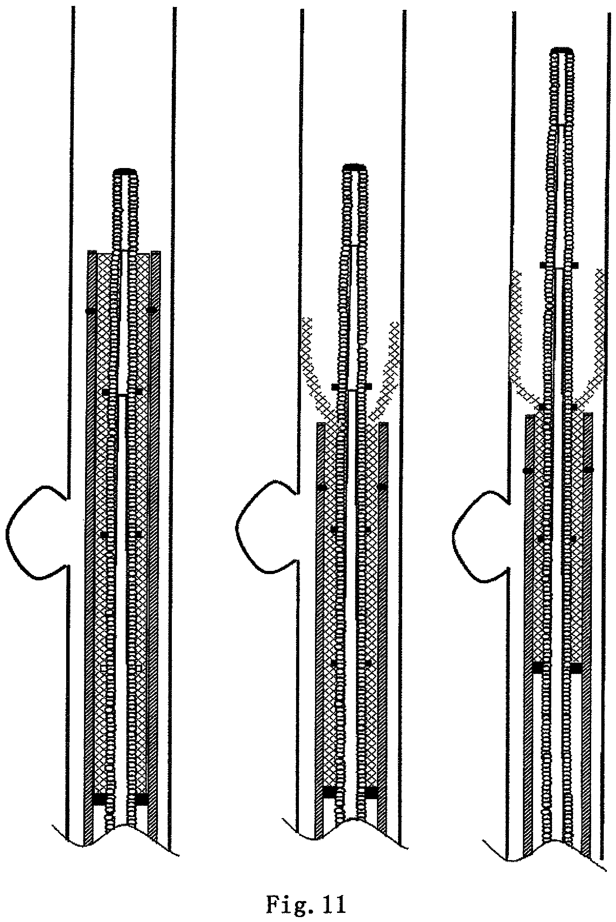

Regarding the process of releasing the stent, as shown in Fig. 10, it can be carried out by pushing the guide wire (2) first so that the frontal end of the stent (1) is released then by withdrawing a segment of the microcatheter (4), and so on and so forth to deploy the stent in a pushing-and-withdrawing way. Also as shown in Fig. 11, the process can be carried out by withdrawing a segment of the microcatheter (4) first so that the frontal end of the stent (1) is released then by pushing the delivery guide wire (2), and so on and so forth to deploy the stent in a withdrawing-and-pushing way. Both methods can release the stent (1) to the site of the vascular lesion. Their difference lies in the distance between the distal end of microcatheter (4) and the aneurysm orifice before the stent (1) is released.

During the release, inaccurate positioning of the stent (1) may happen, so that the stent (1) does not evenly cover the neck of the aneurysm. In this case, the position of the stent (1) can be adjusted in two ways by utilizing the corners of the delivery positioning elements (2-4) on the delivery guide wire (2) which can rub and/or are inserted into the lattices of the stent (1). One way is to keep the position of the delivery guide wire (2) fixed and push the microcatheter (4) slowly to take the stent (1) slowly back into the microcatheter (4) again; the other way is to keep the position of the microcatheter (4) fixed and withdraw the delivery guide wire (2) slowly to bring the stent (1) slowly back into the microcatheter (4) again. After the stent (1) is in the microcatheter (4) again through either way mentioned above, it will be repositioned and redeployed.

In addition, when the stent in the aneurysm surgical apparatus in the examples of the present application is delivered and released to the site of a vascular lesion, it can further serve as a support or a shield for the embolization substance (e.g., a detachable coil, embolic liquid, etc.) in an aneurysm, This will ensure that the embolization material is maintained in the aneurysm only, to keep the parent artery open and to assist the treatment of vascular aneurysm.

The stent of the aneurysm surgical apparatus in the examples of the present application has a high-density lattice structure and thus a high coverage rate. Especially, due to the non-uniform lattice structure on the stent with a high coverage rate adjacent to the aneurysm, it is like that the released stent has reconstructed the arterial wall at the site of the vascular lesion so that the direction of the blood flow at the site can be significantly changed. As a result, blood strikes on the inner wall of the aneurysm have been avoided leading to an achievement of the purpose of the vascular aneurysm treatment. Meanwhile, dense mesh filaments of the stent, serving as a support for the growth or migration of the vascular endothelial cells, accelerate the growth of intima adjacent to an orifice of the aneurysm, so that the blood vessel at the lesion site can be re-covered by intima, thereby achieving a real anatomical cure of aneurysm.

Regarding the aneurysm surgical apparatus in the examples of the present application, the stent is restrained on the delivery guide wire , and the stent and the delivery guide wire are pre-mounted into the introducer sheath. During a surgical delivery, first, the microcatheter is inserted into the pathologically changed blood vessel and then, the introducer sheath is connected to the microcatheter. After that, by applying a force to the delivery guide wire in an axial direction, the stent restrained on the delivery guide wire is fed from the introducer sheath into the microcatheter and moved to the vascular lesion. At the end, the stent is positioned and released at the site of the vascular lesion by adjusting relative positions beween the delivery guide wire and the microcatheter.

## Claims

1. A surgical apparatus for the treatment of an aneurysm, comprising: a stent (1), a delivery guide wire (2), an introducer sheath (3) and a microcatheter (4), wherein:
the stent (1) is self-expanding;
the delivery guide wire (2) is placed in the inner cavity of the introducer sheath (3), with the stent (1) restrained on the outside thereof; and
the introducer sheath (3) is connected with the microcatheter (4), with lumens communicating to form a passageway through which the delivery guide wire (2) and the stent (1) are deliverable into a human body,
wherein the delivery guide wire (2) comprises:
a metal core (2-1) for delivering and supporting the stent (1);
a spring element (2-2) covering the metal core (2-1);
a boss (2-3) fixed on the metal core (2-1), for providing a pushing force for the stent (1) during delivery; and
a plurality of delivery positioning elements (2-4) fixed on the external surface of the spring element (2-2), for providing pushing or withdrawing forces for the stent (1) during delivery.

2. The apparatus according to claim 1, **characterized in that** the self-expanding stent (1) is woven with biocompatible metal filaments and/or polymer filaments (1-1).

3. The apparatus according to claim 2, **characterized in that** the self-expanding stent (1) is in a mesh tube structure.

4. The apparatus according to claim 3, **characterized in that** the mesh tube structure has a compression ratio in a range from 1:2 to 1:10 in the radial direction.

5. The apparatus according to claim 4, **characterized in that** the mesh tube structure is a uniform lattice structure.

6. The apparatus according to claim 5, **characterized in that** the uniform lattice structure has a coverage rate in a range of 20% to 60%.

7. The apparatus according to claim 6, **characterized in that** the uniform lattice structure has a coverage rate in a range of 30% to 50%.

8. The apparatus according to claim 4, **characterized in that** the mesh tube structure as a lattice structure is non-uniform in the axial and/or radial direction at the site of an aneurysm, but is uniform in the rest parts.

9. The apparatus according to claim 8, **characterized in that** the non-uniform lattice structure has a coverage rate in the range of 40% to 60%.

10. The apparatus according to claim 8, **characterized in that** the uniform lattice structure has a coverage rate in a range of 20% to 40%.

11. The apparatus according to claim 1, **characterized in that** materials of the spring element (2-2), the boss (2-3) and the delivery element are visualizable materials.

12. The apparatus according to claim 1, **characterized in that** the introducer sheath (3) is in a hollow structure.

13. The apparatus according to claim 12, **characterized in that** the material of the introducer sheath (3) is a polymeric material.

14. The apparatus according to claim 13, **characterized in that** the polymeric material is PTFE material, HDFE material or FEP material.

15. The apparatus according to claim 1, **characterized in that** the microcatheter comprises:
a tube body (4-1) in a step-like hollow structure with its diameter and hardness decreasing gradually from the proximal end to the distal end;
a stress dispersion tube (4-2) with one end connected with the tube body (4-1) to prevent the tube body from zigzagging at its proximal end; and
an adapting piece (4-3) used to connect the introducer sheath (3) with the tube body (4-1), which is connected with the other end of the stress dispersion tube (4-2) and has the introducer sheath (3) being inserted therein.

16. The apparatus according to claim 15, **characterized in that** the tube body (4-1) is made of the following materials from inside to outside: a polymeric material for a smooth layer, metals and/or polymers for a reinforment layer, and a polymeric material for a jacket layer.

17. The apparatus according to claim 16, **characterized in that** the distal end of the tube body (4-1) is further provided with a visualization element (4-4), for indicating the position of the microcatheter in a blood vessel.

## Patentansprüche

1. Chirurgische Vorrichtung zur Behandlung eines Aneurysmas, die Folgendes umfasst: einen Stent (1), einen Einsetzführungsdraht (2), eine Einführungshülse (3) und einen Mikrokatheter (4), wobei:
der Stent (1) selbstexpandierend ist;
der Einsetzführungsdraht (2) in dem inneren Hohlraum der Einführungshülse (3) platziert ist, wobei der Stent (1) auf dessen Außenseite zurückgehalten ist; und
die Einführungshülse (3) mit dem Mikrokatheter (4) verbunden ist, wobei Lumen kommunizieren, um einen Durchgang zu bilden, durch den der Einsetzführungsdraht (2) und der Stent (1) in einen menschlichen Körper einsetzbar sind,
wobei der Einsetzführungsdraht (2) Folgendes umfasst:
einen Metallkern (2-1) zum Einsetzen und Stützen des Stents (1);
ein Federelement (2-2), das den Metallkern (2-1) bedeckt;
einen Vorsprung (2-3), der auf dem Metallkern (2-1) befestigt ist, um eine Schubkraft für den Stent (1) während des Einsetzens bereitzustellen, und
eine Vielzahl von Einsetzpositionierungselementen (2-4), die auf der äußeren Oberfläche des Federelements (2-2) befestigt sind, um Schub- oder Rückzugkräfte für den Stent (1) während des Einsetzens bereitzustellen.

2. Vorrichtung nach Anspruch 1, **dadurch gekennzeichnet, dass** der selbstexpandierende Stent (1) mit biokompatiblen Metallfäden und/oder Polymerfäden (1-1) geflochten ist.

3. Vorrichtung nach Anspruch 2, **dadurch gekennzeichnet, dass** der selbstexpandierende Stent (1) eine Maschenröhrenstruktur ist.

4. Vorrichtung nach Anspruch 3, **dadurch gekennzeichnet, dass** die Maschenröhrenstruktur ein Kompressionsverhältnis in einem Bereich von 1:2 bis 1:10 in die radiale Richtung hat.

5. Vorrichtung nach Anspruch 4, **dadurch gekennzeichnet, dass** die Maschenröhrenstruktur eine gleichmäßige Gitterstruktur ist.

6. Vorrichtung nach Anspruch 5, **dadurch gekennzeichnet, dass** die gleichmäßige Gitterstruktur eine Deckungsrate in einem Bereich von 20 % bis 60 % hat.

7. Vorrichtung nach Anspruch 6, **dadurch gekennzeichnet, dass** die gleichmäßige Gitterstruktur eine Deckungsrate in einem Bereich von 30 % bis 50 % hat.

8. Vorrichtung nach Anspruch 4, **dadurch gekennzeichnet, dass** die Maschenröhrenstruktur als eine Gitterstruktur in die axiale und/oder radiale Richtung an der Stelle eines Aneurysmas nicht gleichmäßig ist, aber in den restlichen Teilen gleichmäßig ist.

9. Vorrichtung nach Anspruch 8, **dadurch gekennzeichnet, dass** die nicht gleichmäßige Gitterstruktur eine Deckungsrate in dem Bereich von 40 % bis 60 % hat.

10. Vorrichtung nach Anspruch 8, **dadurch gekennzeichnet, dass** die gleichmäßige Gitterstruktur eine Deckungsrate in einem Bereich von 20 % bis 40 % hat.

11. Vorrichtung nach Anspruch 1, **dadurch gekennzeichnet, dass** Materialien des Federelements (2-2), des Vorsprungs (2-3) und des Einsetzelements visualisierbare Materialien sind.

12. Vorrichtung nach Anspruch 1, **dadurch gekennzeichnet, dass** die Einführungshülse (3) eine Hohlstruktur ist.

13. Vorrichtung nach Anspruch 12, **dadurch gekennzeichnet, dass** das Material der Einführungshülse (3) ein Polymermaterial ist.

14. Vorrichtung nach Anspruch 13, **dadurch gekennzeichnet, dass** das Polymermaterial PTFE-Material, HDFE-Material oder FEP-Material ist.

15. Vorrichtung nach Anspruch 1, **dadurch gekennzeichnet, dass** der Mikrokatheter Folgendes umfasst:
einen Röhrenkörper (4-1) mit einer stufenähnlichen Hohlstruktur, deren Durchmesser und Härte allmählich von dem proximalen Ende zu dem distalen Ende abnehmen;
eine Belastungsdispersionsröhre (4-2) mit einem Ende, das mit dem Röhrenkörper (4-1) verbunden ist, um zu verhindern, dass der Röhrenkörper an seinem proximalen Ende eine Zickzackbewegung ausführt, und
ein Anpassteil (4-3), das verwendet wird, um die Einführungshülse (3) mit dem Röhrenkörper (4-1) zu verbinden, der mit dem anderen Ende der Belastungsdispersionsröhre (4-2) verbunden ist und in den die Einführungshülse (3) eingeführt ist.

16. Vorrichtung nach Anspruch 15, **dadurch gekennzeichnet, dass** der Röhrenkörper (4-1) von innen nach außen aus den folgenden Materialien gebildet ist: ein Polymermaterial für eine glatte Schicht, Metalle und/oder Polymere für eine Verstärkungsschicht und ein Polymermaterial für eine Mantelschicht.

17. Vorrichtung nach Anspruch 16, **dadurch gekennzeichnet, dass** das distale Ende des Röhrenkörpers (4-1) ferner mit einem Visualisierungselement (4-4) versehen ist, um die Position des Mikrokatheters in einem Blutgefäß anzugeben.

## Revendications

1. Instrument chirurgical destiné au traitement d'un anévrisme, comprenant: un stent (1), un fil guide de pose (2), une gaine d'introduction (3) et un microcathéter (4), dans lequel :
le stent (1) est auto-expansible ;
le fil guide de pose (2) est placé dans la cavité interne de la gaine d'introduction (3), le stent (1) étant retenu sur l'extérieur de celui-ci ; et
la gaine d'introduction (3) est reliée au microcathéter (4), avec les lumières communiquant pour former un passage à travers lequel le fil guide de pose (2) et le stent (1) peuvent être installés dans un organisme humain,
le fil guide de pose (2) comprenant :
une âme métallique (2-1) pour installer et supporter le stent (1) ;
un élément élastique (2-2) recouvrant l'âme métallique (2-1) ;
un bossage (2-3) fixé sur l'âme métallique (2-1), afin de fournir une force de poussée pour le stent (1) au cours de la pose ; et
une pluralité d'éléments de positionnement pour la pose (2-4) fixés sur la surface externe de l'élément élastique (2-2), afin de fournir des forces de poussée ou de retrait pour le stent (1) au cours de la pose.

2. Instrument selon la revendication 1, **caractérisé en ce que** le stent auto-expansible (1) est tissé avec des filaments de métalliques et/ou des filaments polymères (1-1) biocompatibles.

3. Instrument selon la revendication 2, **caractérisé en ce que** le stent auto-expansible (1) est une structure tubulaire maillée.

4. Instrument selon la revendication 3, **caractérisé en ce que** la structure tubulaire maillée présente un rapport de compression situé dans la plage allant de 1/2 à 1/10 dans le sens radial.

5. Instrument selon la revendication 4, **caractérisé en ce que** la structure tubulaire maillée est une structure uniforme de type treillis.

6. Instrument selon la revendication 5, **caractérisé en ce que** la structure uniforme de type treillis présente un taux de couverture situé dans une plage allant de 20 % à 60 %.

7. Instrument selon la revendication 6, **caractérisé en ce que** la structure uniforme de type treillis présente un taux de couverture situé dans une plage allant de 30 % à 50 %.

8. Instrument selon la revendication 4, **caractérisé en ce que** la structure tubulaire maillée, telle qu'une structure de type treillis, n'est pas uniforme dans le sens axial et/ou radial au niveau du site d'un anévrisme mais est uniforme dans les parties restantes.

9. Instrument selon la revendication 8, **caractérisé en ce que** la structure non uniforme de type treillis présente un taux de couverture situé dans une plage allant de 40 % à 60 %.

10. Instrument selon la revendication 8, **caractérisé en ce que** la structure uniforme de type treillis présente un taux de couverture situé dans une plage allant de 20 % à 40 %.

11. Instrument selon la revendication 1, **caractérisé en ce que** les matériaux de l'élément élastique (2-2), le bossage (2-3) et l'élément de pose sont des matériaux visualisables.

12. Instrument selon la revendication 1, **caractérisé en ce que** la gaine d'introduction (3) est une structure creuse.

13. Instrument selon la revendication 12, **caractérisé en ce que** le matériau de la gaine d'introduction (3) est un matériau polymère.

14. Instrument selon la revendication 13, **caractérisé en ce que** le matériau polymère est un matériau de type PTFE, un matériau de type HDFE ou un matériau de type FEP.

15. Instrument selon la revendication 1, **caractérisé en ce que** le microcathéter comprend :
un corps tubulaire (4-1) sous forme de structure creuse échelonnée dont le diamètre et la dureté diminuent progressivement de l'extrémité proximale jusqu'à l'extrémité distale ;
un tube de dispersion de contraintes (4-2) dont une extrémité est reliée au corps tubulaire (4-1) afin d'empêcher le corps tubulaire de zigzaguer au niveau de son extrémité proximale ; et
un adaptateur (4-3) utilisé pour relier la gaine d'introduction (3) au corps tubulaire (4-1), qui est relié à l'autre extrémité du tube de dispersion de contraintes (4-2) et dans lequel est insérée la gaine d'introduction (3).

16. Instrument selon la revendication 15, **caractérisé en ce que** le corps tubulaire (4-1) se compose, de l'intérieur vers l'extérieur, des matériaux suivants : un matériau polymère pour une couche lisse, des métaux et/ou des polymères pour une couche de renfort et un matériau polymère pour une couche de type chemise.

17. Instrument selon la revendication 16, **caractérisé en ce que** l'extrémité distale du corps tubulaire (4-1) est en outre munie d'un élément de visualisation (4-4), afin d'indiquer la position du microcathéter dans un vaisseau sanguin.
